# EUROPEAN PATENT SPECIFICATION

(11) **EP 2 502 986 B1**
(45) Date of publication and mention of the grant of the patent: **26.06.2019**
(21) Application number: 11159252.3
(22) Date of filing: 22.03.2011
(51) Int. Cl.: C12N 5/07, C12M 3/00, C12M 3/08

(54) **A cartilage cell processing system**
Knorpelzellverarbeitungssystem
Système de traitement cellulaire de cartilage

(43) Date of publication of application: 26.09.2012
(73) Proprietor: CartiRegen B.V., 3971 NG Driebergen-Rijsenburg (NL)
(72) Inventor: Hendriks, Jeanine Anna Alphose, 3702 SC Zeist (NL); Riesle, Jens Uwe, 3761 EG Soest (NL); Wilson, Clayton Ellis, 3707 SE Zeist (NL); van den Doel, Mirella Ageeth, 2906 RA Capelle a/d Ijssel (NL)
(74) Representative: V.O.

(56) References cited:
- WO-A2-03/087292
- WO-A2-2008/082829
- JP-A- 2008 212 019
- US-A1- 2008 113 426
- US-A1- 2010 112 696

## Description

### FIELD OF THE INVENTION

The invention relates to a module-based cartilage cell processing device for preparing a cell seeded carrier from a biological material.

The invention still further relates to cartilage preparation module for use in a cartilage cell processing device.

The invention still further relates to a method of cartilage cell processing.

### BACKGROUND OF THE INVENTION

Primary cells are highly specialized cells present in the various specific types of tissue in an organism. They are involved in maintaining, repairing and supporting the function of said tissue. In most situations where a defect occurs in living tissue, some intrinsic or extrinsic reaction is triggered. The primary cells that are present in the damaged tissue may produce specific growth and other factors which will be secreted to the surroundings of the defect. This is aimed at triggering proliferation of the still viable cells whereby the defect may become filled. Next, if necessary, the cells may differentiate into the required cell type to produce and maintain fully functional specialized tissue. In many cases, however, the repair reaction of the body is not or not fully leading to a functional tissue. This may be due to a variety of reasons, such as the size of the defect, the poor availability of primary cells at the site of the defect to support the repair function, or the lack of influx from multipotent cells, which may differentiate to the required cell type, e.g. via the blood stream. Articular cartilage covers the ends of long bones of synovial joints and consists of approximately 30% of extracellular matrix proteins and approximately 70% water. Chondrocytes are the only cell type found in normal articular cartilage but contribute less then 2% of the wet weight in human healthy adult tissue. The extracellular matrix consists predominantly of cartilage specific proteoglycan molecules with highly negatively charged sulfated glycosaminoglycan (GAG side chains, as well as type II collagen fibrils. The GAG side chains are able to bind water molecules, thereby sequestering water and generating an internal swelling pressure within the cartilage matrix. These hydrogel-like properties are essential for the interstitial fluid flow patterns observed inside the matrix during functional loading of cartilage, at which point water is forced out of the tissue to an amount that allows the negatively charged GAG chains to repel each other. Upon release of the compressive load, water is imbibed back into the tissue matrix. The collagenous network, together with water bound GAG, enables articular cartilage to withstand large compressive loads which gives the tissue its unique function in synovial joints: smooth and pain-free articulation, spreading of the applied load onto the subchondral bone and absorbing mechanical shocks. Mature articular cartilage matrix is neither vascularized nor innervated, containing chondrocytes at low numbers which do not divide after skeletal maturity. It is partly for this reason that articular cartilage does not repair spontaneously or only to a very limited extent. Current approaches for cartilage repair rely on removal of tissue debris, access to the wound healing system of bone by penetrating the subchondral bone plate, and tissue transplantation and cell based therapies. Current clinical therapies are limited to autologous cell based therapies, such as autologous chondrocytes implantation (ACI) and mosaicplasty (also known as autologous osteochondral grafts). Due to severe drawbacks, both therapies can currently only address a limited share of the cartilage repair market. For mosaicplasty, a major disadvantage is the limitation to small defects due to limited availability of donor tissue for transplantation. For ACI, drawbacks include the necessity to perform two surgical operations, high costs due to the required culturing of cells in vitro, and loss of phenotype of cartilage cells. Cartilage cells de-differentiate upon cell expansion, which is part of the ACI process. Hence, they require several months after surgery before they regain their original phenotype. Only then true cartilage repair can commence. Recently, a second generation ACI has been developed involving autologous chondrocytes in a biomaterial matrix. This technique solves some of the problems of ACI, particularly the long and open surgical procedure that was required in ACI. However, three important drawbacks remain: two surgical procedures have to be carried out, high costs and long rehabilitation. Accordingly, there is a need for further improvements in the field of repair of tissue defects, in particular for defects which are not, or not sufficiently repaired in a spontaneous fashion.

WO 2008/082829 discloses a hyaline-like, single layer cartilage tissue construct that includes chondrogenic cells dispersed within an endogenously produced extracellular matrix. The single layer cartilage tissue construct has a glycosaminoglycan content substantially equal to the glycosaminoglycan content of native cartilage tissue. Also method is disclosed for generating a single layer cartilage tissue construct that includes isolating a population of chondrogenic cells and then expanding the population of chondrogenic cells. Next, the population of chondrogenic cells is seeded into a bioreactor having a volume defined by oppositely disposed gas permeable membranes.

JP2008212019 discloses a device for decomposing the living body tissue. The device is equipped with a container for storing digestive liquid containing a digestive enzyme for decomposing the living body tissue, and an agitating blade, which is immersed in the digestive liquid stored in the container. The agitation blade agitates the digestive liquid by being relatively moved with respect to the container. The device further comprises a driving device for driving the agitating blade and a decomposition state-determining part for determining the progressing state of the decomposition of the living body tissue. The driving device is equipped with a driving source and an elastic body connecting the driving source to the agitating blade. The decomposition state-determining part determines the progressing state of decomposition of the living body tissue, based on the delay of movement of the agitating blade relative to the movement of the driving source.

### SUMMARY OF THE INVENTION

It is an object of the invention to provide an efficient cell processing device. More in particular, it is an object of the invention to provide cell processing device capable of enabling a single-surgery treatment of cartilage defects, for example in a knee joint. Still more in particular, it is an object of the invention to provide a cartilage cell processing device wherein custom modification either of its hard-ware architecture and/or of the mode of operation is possible.

To this end in accordance with a first aspect of the invention an automated or semi-automated module-based cartilage cell processing device for preparing a cell seeded carrier from a biological material is provided, said device comprising at least a cartilage preparation module, a cell isolation module, a cell mixing module and a carrier cell seeding module. In this device, the cell mixing module and the carrier cell seeding module are integrated to provide a single unit and the cartilage preparation module comprises a blender comprising a blade which is rotatable in two directions, each rotating direction causing a substantially unique effect of the blade on the cartilage cells.

It will be appreciated that in an embodiment only cartilage cells may be processed. Alternatively, the device according to the invention may function to allow processing of the cartilage cells and combining them with crude bone marrow, mononucleated cells from bone marrow or multipotent cells from bone marrow or other tissues prior to seeding. For the suitable carrier either scaffold or gel may be used.

It is found that substantial clinical benefits can be provided for patients when the module-based semi-automatic or fully automatic cartilage cell processing device according to the foregoing is used during a cartilage repair intervention, as the single-surgery treatment of the knee cartilage can be enabled.

An embodiment of a general cell processing device is known from WO 2006-051538. In the known device stem cells are harvested, in particular from mesenchymal and-or hematopoietic stem cells. In accordance with the known device a suitable soft tissue mass, for example a placenta or an umbilical cord are placed in a sealable container provided with an organ disrupter arranged for disrupting a structure of the organ thereby yielding a cell suspension. The disrupter is arranged as a rotatable, a translatable or a vibratable body.

A further embodiment of a cell processing device is known from US 2007-0148756. In the known device a cell suspension may be prepared. For this purpose the device comprises a movable dissociation element arranged inside a container for engaging a tissue for causing said dissociation. The dissociation element may be implemented as a blade, a serrated member, a member having surface roughness, a rotating strand, or a member having the 3D dissociation features. The blades of the known dissociation device may be triangular in shape having beveled cutting edges. The blade of the known dissociation device may be rotated with a relatively slow speed for merely distributing the tissue inside the container rather than for cutting it.

Due to the fact that the module-based cartilage cell processing device according to the invention comprises a number of separate modules, which may be removable and/or disposable, a custom automated or semi-automated cell processing system can be built having a flexible architecture. As a result, a suitable biological material can be fully automatically processed in the cartilage cell processing device according to the invention so that the cell seeded carrier, like scaffold, gel or other implantable biomaterial, can conveniently be prepared maintaining sterility. It is found to be advantageous to provide disposable modules, however, it is also possible to provide a suitable portion of the device as an reusable item. It will be appreciated, however, that using closed, preferably aseptic, disposable modules is advantageous as manual handling of the biological material between different separated modules is avoided improving sanitary quality of the procedure as a whole. However, a semi-automatic mode is also allowed, for example, when the transfer is carried out using a luerlock or a similar interface.

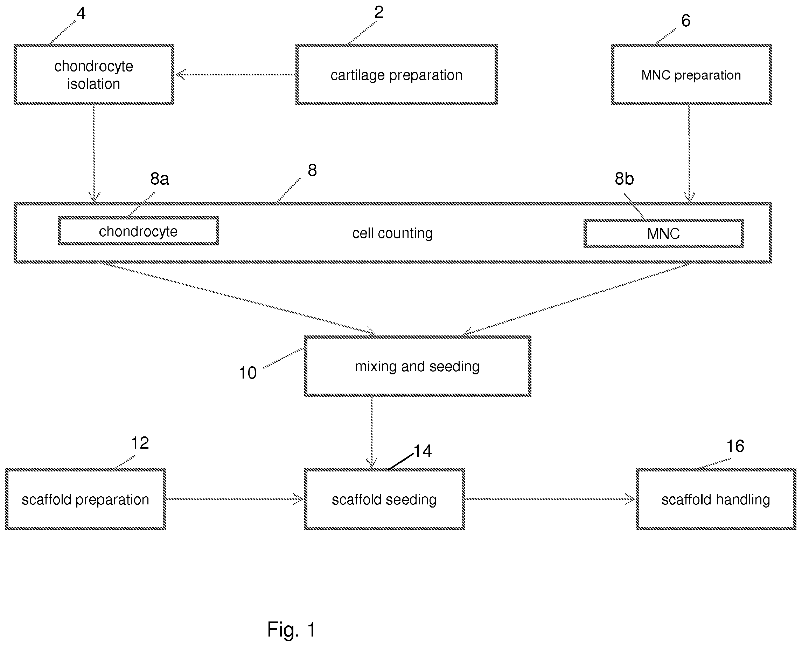

In general, by way of example, the process of scaffold preparation may be seen as follows. Suitable cartilage may be collected and cut, minced or mechanically processed in a different way to form a chopped cartilage. The chopped cartilage may be further processed for chondrocyte isolation using, for example, a process of digestion followed by a process of isolation and washing of the desired cells. The thus formed and prepared biological material, which may contain a suitable buffered cell suspension, may be counted together with suitable cells (mononuclear cells and/or multipotent cells) which may be prepared using filtering red blood cells, removing plasma and washing the remaining cells. It will be appreciated that the mononuclear cells and/or multipotent cells may be derived from tissue, bone marrow or from any other suitable source. After the cells are counted the biological material may be mixed, whereby the cell quantity may additionally be determined before the step of mixing. After the step of mixing is completed, the cells may be seeded on a prepared scaffold, which may be further sized or otherwise processed prior to implanting. More details on this process will be provided with reference to Figure 1.

It is found to be particularly advantageous to integrate the mixing step and the carrier cell seeding step in one module. As a result no transport of biological material is required and the accuracy of both the mixing and the seeding may be increased.

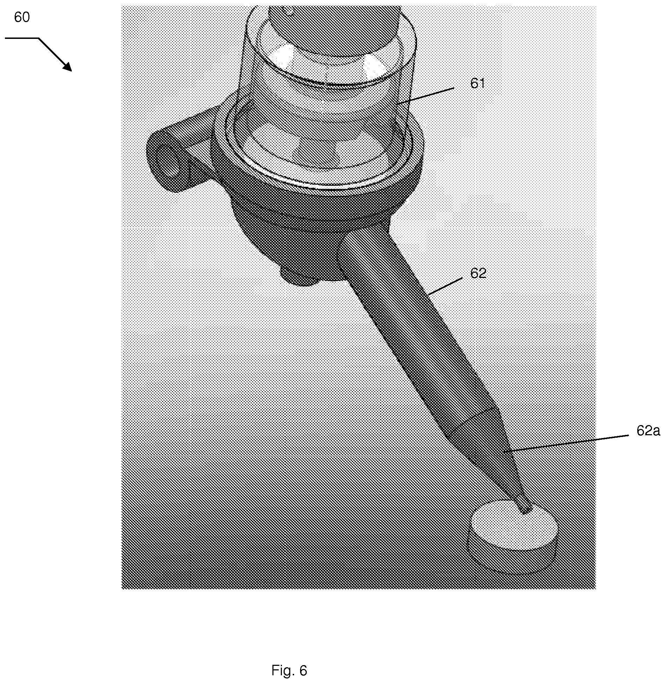

For example, by using a suitable reservoir may be used for mixing/seeding which collects cells from a suitable module of the device according to the invention after the cells have been released. The reservoir may achieve a homogeneous population of cells. More details on this embodiment will be discussed with reference to Figure 6.

Preferably, the cutter is a shear cutter comprising a blade. However, a plurality of blades may be used. The blade may be manufactured from a plastics material. However, metal may be used as well. The blender comprises a blade which is rotatable in two directions. The blade causes a substantially unique effect on the cartilage cells present in the blender. For example, a first rotating direction may be used for exerting shear forces on the biological material and the second direction may be used for massaging and pressing the biological material. This has an advantage of enabling preparation of a substantially homogeneous biological mass. However, the cutter may be adapted to push cartilage through a grid of blades or cutting wires. The blender is further adapted to combine a cutting step with a gyration or whirling step.

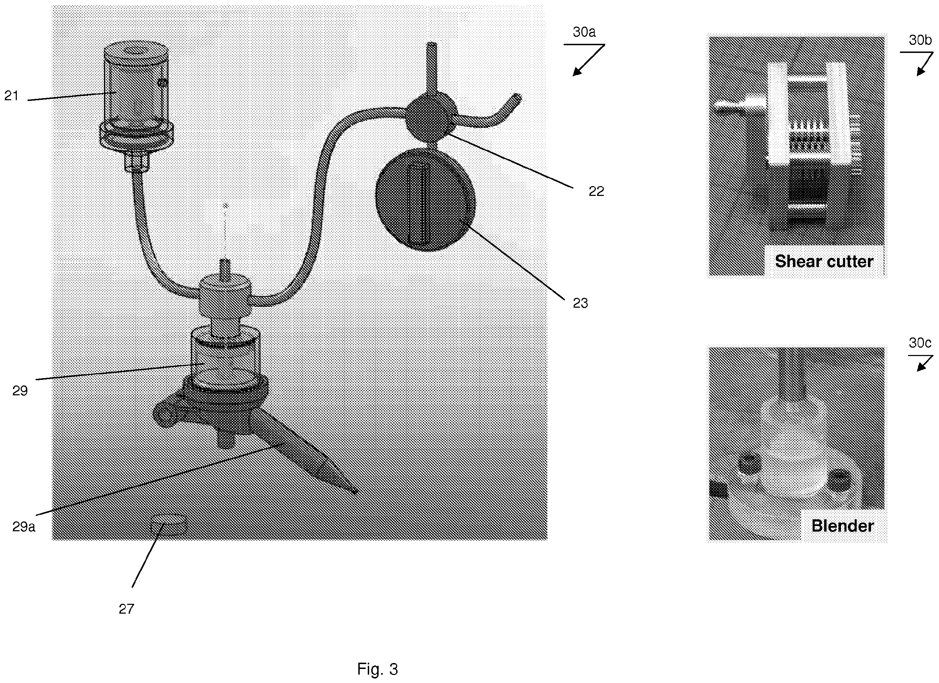

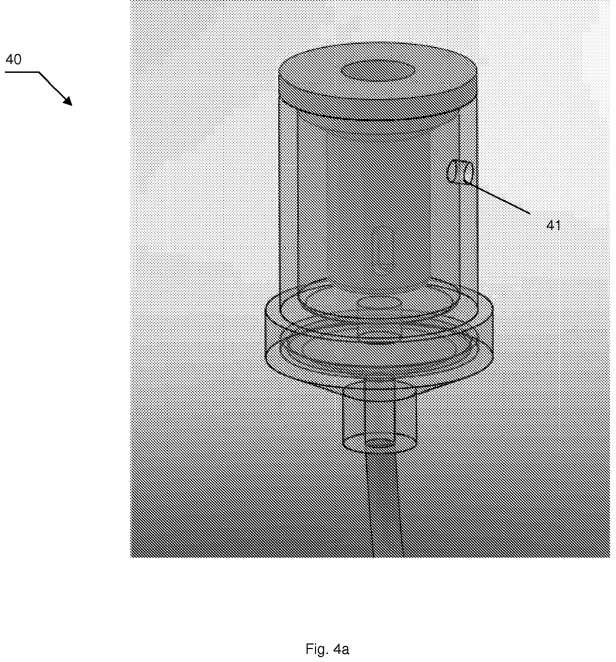

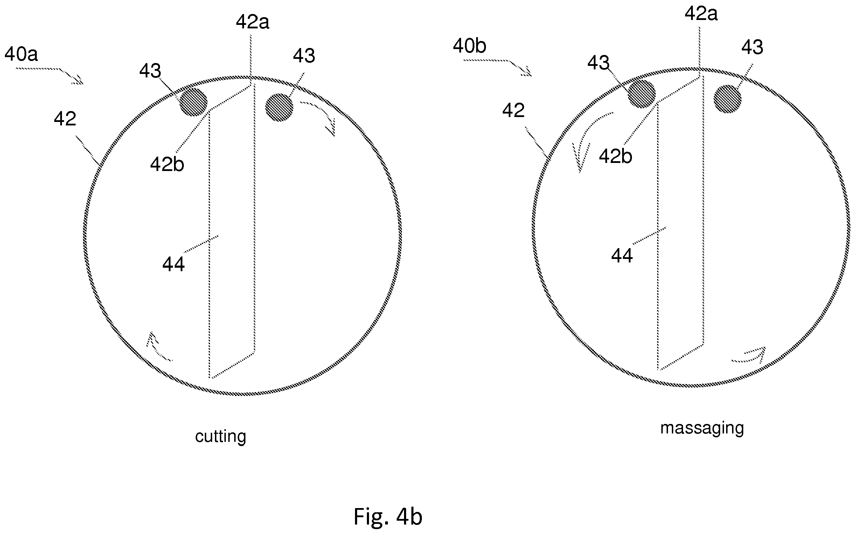

As a result the blender is adapted to combine a cutting step with a mixing or gyration and whirling steps. More details on these embodiments will be given with reference to Figures 3 and 4.

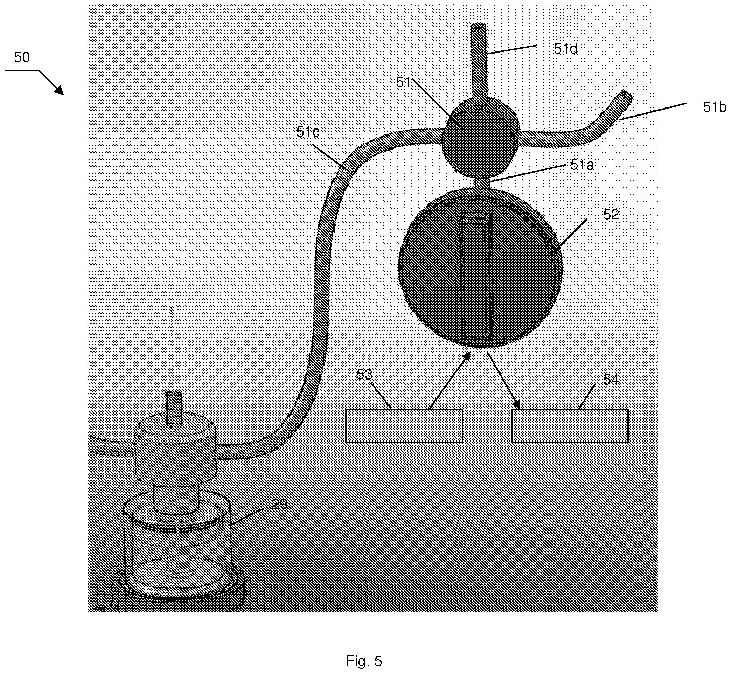

In a still further embodiment of the cartilage cell processing device the cell isolation module comprises a filter having a release buffer and/or a centrifugation unit. An embodiment of the cell isolation module is discussed with reference to Figure 5.

Preferably, the counter is operable using a principle selected from: a bulk through flow, a slide sample or a sample through flow. It will be appreciated that a suitable counting module may be used for counting of the isolated cells and/or for counting of the chondrocyte cells. Preferably, the counted is adapted to be suitable for counting both the chondrocyte cells and the cells. The counting may be performed within a relatively short period of time, less than a minute, for example.

The bulk through flow counting method may be used in the cartilage cell processing device according to the invention. Such counting may be carried out when the cells pass through a precision orifice, which may be less than 0.1 mm in diameter. The restriction forces the cells into a single file. It may be preferable that a passage used for accommodating the cell flow is adapted for causing a laminar flow. This measuring method may be based on a variance in the electrical impedance of an electrolyte comprising particles as they pass through the orifice. Still another method of measuring may be base on optical properties of a suitable light beam, such as a laser, impinging on the cells which are forced into a single file. The laser beam may cause hydrodynamic focusing. When the cells pass in a laminar flow and intercept the laser beam the light is scattered, which may be monitored by a suitable detector.

Slide sample counting method may be used as an alternative. However, it will be appreciated that slide based systems may be destructive and may require additional strains or fluorescent dyes, but can perform measurements which are quick are reproducible. The volume of sample required for carrying out such counting is about 20 micro liter. The optimum total number of cells required for a test may be dependent on the desirable speed of counting. Preferably, about 40 cells per quadrant may be used at a sample volume of 20 micro liter yielding a minimum sample concentration of 6000 cells/ml.

Sample through flow method may be still further suitable embodiment of a counting method used in the cartilage cell processing device according to the invention. This method requires preparation of a representative sample of the cell solution in terms of cell concentration and volume, adding the required dyes or fluorescent tags and passing it through a counting and viability device, such as a flow cytometer. This method usually utilized fluorescent antibody tags on cells which are sorted by passing cells through a suitable nozzle adapted to break the stream of cells into drops. A charge is applied to the thus formed drops and they are sorted by an electric field.

It is also possible to count cells using an image analysis of a suitably stained slide.

In a further advantageous embodiment the carrier cell seeding module is integrated with a mixing unit. Preferably, it comprises at least a partially disposable syringe or a concentration tip. More detail on this embodiment will be presented with reference to Figure 6.

In a still further embodiment of the cartilage cell processing device according to the invention the carrier cell seeding module is arranged to measure a volume of the cell suspension in order not to overflow the carrier using a measuring device. A counter and a measuring device may be used for two different purposes:
i) to determine the cell count;
ii) to determine the volume of cell suspension which maximally will be added to the chondrocyte cell suspension.

This feature is found to be advantageous, because the accuracy of the automated carrier seeding may be improved to a further extent. For example, when the system detects that there is not enough biological material for preparing a desired carrier cell seeding, a warning signal may be given signaling that the counting number of a suitable matter, for example, chondrocytes is not optimal.

It will be appreciated that in order to improve sterility requirements regarding the scaffold preparation procedure, at least one of the modules of the cartilage cell preparation device may comprise suitable antibiotics. However, alternatively, the solutions used in the module-based device according to the invention may be provided with antibiotics instead.

In a further embodiment, wherein the biological material comprises red blood cells, the cell isolation module is operable using a method to lyse the red blood cells, preferably using NH₄Cl.

For, example the mononuclear cells may be isolated from red blood cells using the following steps:
1. measure bone marrow volume and transfer the bone marrow aspirate to a sterile 125 ml or 500 ml bottle, (Whole Bone Marrow, further referred to as WBM).
2. if required remove whole bone marrow sample before further processing (e.g. cell culturing, seeding of WBM, etc.).
3. if bone marrow plasma is required continue with steps of centrifuging bone marrow for 20 minutes, 300g at room temperature;
4. lyse cells by diluting 50 x using red blood cell lysing buffer (example: add to 1 ml of bone marrow 49 ml of red blood cell lysing buffer).
5. mix by pipetting up & down 10 times and transfer to 50 ml tubes for incubation at RT until the liquid appears clear red (approximately 5-30 min).
6. centrifuge for 2 min at 750 g, RT.
7. discard the supernatant, containing the lysing buffer and lysed cells.
8. wash cells by resuspending the cell pellet in total 50 ml medium (e.g. RPMI or DMEM, containing PenStrep), per tube and centrifuge 2 min at 750 g, RT.
   discard supernatant and resuspend cell pellets in total 10 - 30 ml medium + PenStrep. Volume may dependon WBM count: for example if a lot
9. Count cells;
   - dilute 10x by pipetting 50 µl WBM + 450 µl red blood cell lysing buffer, incubate for 5 to 10 min;
   - take 20 µl lysed cells + 20 µl trypan blue and count: when difficult to count because of high number of red blood cells repeat this step with a new dilution: prepare a 50 x dilution (20 µl WBM + 980 µl red blood cell lysing buffer);
10. keep the cells at room temperature until further processing the cells.

The invention still further relates to a cartilage preparation module for use in a module-based cartilage cell processing device, wherein the cartilage preparation module comprises a blender as defined in claim 16.

A method of cartilage cell processing, according to the invention, comprises the following sequence of automated steps:
- cartilage cell preparation;
- cell isolation;
- cell mixing using the prepared cartilage cells and the isolated cells ;
- Cell seeding on a carrier using said mixture
wherein cell mixing and cell seeding is carried out in an automated or semi-automated module-based cartilage cell processing device as defined in claim 1.

For the carrier either a scaffold, gel or other implantable carrier may be used. Suitable examples of the scaffold, gel or other implantable carrier are poly (ethylene glycol) terepthalate (PEGT) and poly (butylene terephtalate (PBT) polymers, collagen scaffold or gel, esterified hyaluronic acid scaffolds, fibrin glue, alginate, hyaluronic acid, PLGA, PEG based scaffolds or gels, Polyacrylamide carriers, Dextran based carriers like cross-linked Dextran-tyramine hydrogel and self assembly based gels, and derivatives thereof. It will be appreciated that the above examples are neither limiting nor exhaustive. It will be further appreciated that further examples of suitable carriers which may be practiced with the present invention are described in a patent publication WO2005/087239 of the Applicant.

These and other aspects of the invention will be further discussed with reference to drawings wherein like reference numbers refer to like elements. It will be appreciated that the drawing are provided for illustrative purposes only and may not be used for limiting the scope of the appended claims.

### BRIEF DESCRIPTION OF THE DRAWINGS

Figure 1 presents in a schematic way a block-scheme of processing steps as may be used in the cartilage cell processing device according to the invention.
Figure 2 presents a schematic view of the overall architecture of the cartilage cell processing device according to the invention.
Figure 3 presents in a schematic way an embodiment of a shear cutter which may be used in the cartilage cell processing device according to an aspect of the invention.
Figure 4a presents a schematic view of an embodiment of a blender which may be used in the cartilage cell processing device according to the invention.
Figure 4b presents a schematic view of an embodiment of a blender blade which may be used in the cartilage cell processing device according to the invention.
Figure 5 presents schematically an embodiment of the MNC module for use in the cartilage cell processing device according to the invention.
Figure 6 presents in a schematic way an embodiment of a scaffold cell mixing and seeding device.

### DETAILED DESCRIPTION OF THE DRAWINGS

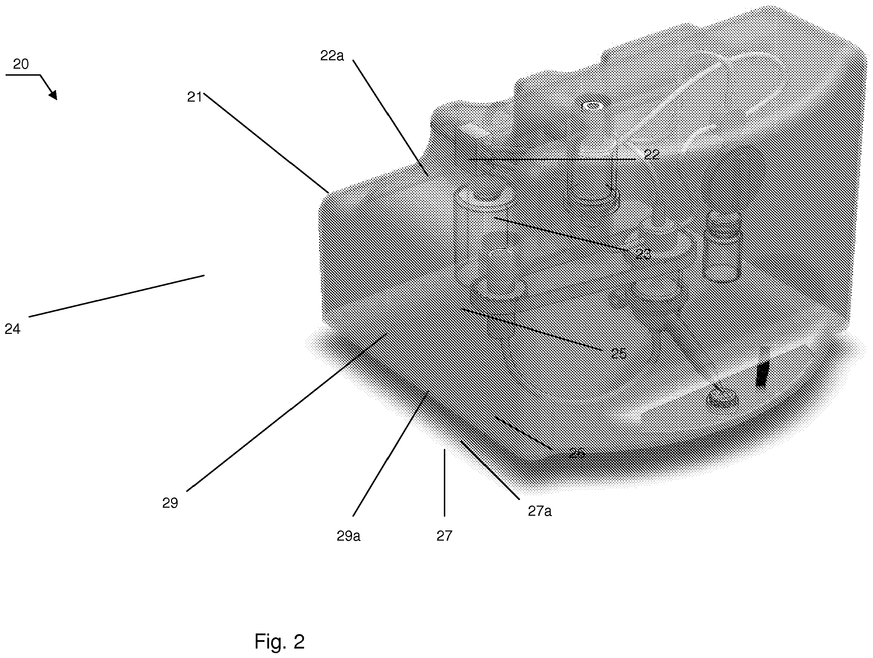

Figure 1 presents in a schematic way a block-scheme of processing steps as may be used in the cartilage cell processing device according to the invention. In this embodiment one of many possible cell processing steps using the device of the invention shown in Figure 2 will be explained.

At step 2 cartilage preparation may be carried out. It will be appreciated that cartilage cutting may be critical to the following process parameters:
- the size of the cartilage pieces influencing a speed with which chondrocytes are liberated;
- the viability of the cells may be sensitive to the processing conditions, such as size of cut cartilage pieces and the cutting process;
- the quality of cells may be sensitive to the processing conditions, such as size of cut cartilage pieces and the cutting process.

It is found that an automated cutting using a suitable cutting device is beneficial for producing high yield of cells having good quality. Plastics cutting blades, which may be used in a shear cutter or blender may be advantageous. It is found that a shear cutter is capable of producing pieces of cartilage with a substantially constant size.

A blender is used in the claimed invention for preparing the cartilage pieces at step 2 of Figure 1. The blender may be advantageous as it is quicker than the shear cutter. The blender may comprise a blade which is manufactured from a e.g. plastics or metal material. A shape of the blade may be adapted to be non-planar, for example to be propeller-shaped. In addition, the blade is arranged to be rotatable in two directions, wherein in the first direction the cartilage will be cut and in the second direction the cells will be massaged yielding a substantially homogenized mass.

Another advantage in using the blender is that it may be integrated with mixing of collagenase and/or with a digestion step. Preferably, the blender is sealed which is beneficial in terms of sterility. The blender may be adapted to provide both cutting and vortexing, which may advantageous reduce the digestion time under 30 - 45 minutes, or so.

It will be appreciated that the step 2 as is described with reference to the foregoing may be carried out using a dedicated hardware outside the device according to the specification as depicted in Figure 2.

At step 4 chondrocyte isolation is carried out, in a module of the automated device according to the invention. The chondrocyte isolation step may be accompanied by a digestion and a washing step. It is found that chondrocyte isolation may be sensitive to isolation conditions which may affect cell numbers, cell viability, cell quality and digestion time. It is found to be preferable to carry-out the step of isolation at temperature of 37 degrees Celsius, using a buffer medium, such as MEM or RPMI, and an optimized mixing mode.

At step 8 the chondrocyte cells may be counted using a suitable counting method 8a, as is described with reference to the foregoing.

Parallel to steps 2 - 8, it is possible to carry out the step 6 of MNC preparation, preferably using a dedicated MNC preparation module. The user may add bone marrow biopsy, any required fluids to the MNC module to process the MNC cells in, for example, RPMI.

The step 6 may include the additional steps, such as the step of lysing or filtering out red cells; separating MNC from plasma, lysing with a suitable buffer, etc. Also cell washing may be envisaged as well as adding a suitable cell buffer, such as RPMI.

The isolated MNC is followed to the counting stage at step 8, wherein the MNC yield is counted using a counter 8b. Preferably, the counting method used for chondrocyte is the same as the counting method used for the MNC cells so that a universal counting stage may be used. This has an advantage of simplifying the architecture of the overall device.

At step 10 after the cells are counted, the cell mixing is taking place. For example, all chondocytes may be added at this step followed by an additional measurement of the quantity of MNC. After the cells are suitably mixed, the excess cell buffer, such as RPMI may be removed.

The step 10 is followed by the step 14 of cell seeding on a scaffold preparation provided at step 12. It will be appreciated that particular advantages are provided when the steps of mixing and seeding are carried out using the same hardware module.

After the prepared scaffold is seeded, the resulting biological material may be further processed, for example it may be suitably sized and implanted.

Figure 2 presents a schematic view of the overall architecture of the cartilage cell processing device according to the invention. The device 20 according to the invention is adapted to allow a continuous transport of the biological material between the different modules of the device in an automated way. It will be appreciated that the modules may be disposable. It will be further appreciated that at least some of the modules may be integrated.

The device 20 comprises a cartilage preparation module 21 comprising a cutter/digester unit, a fluid supply port for supplying an enzyme or a washing fluid. The cartilage preparation module may have an internal volume of about 5 ml. An exit port of the cartilage preparation module 21 may be provided with a suitable filter.

It this embodiment the device 20 further comprises an MNC module 22 comprising a suitable isolation unit, such as a filter 23 and a pump. The MNC module 22 is connected via a conduit 22a with the mixing/seeding module 29 provided with a syringe 29a.

The MNC module 22 may be further provided with a waste reservoir 25 for collecting plasma, for example. The MNC module may be operable using an affinity filter or using a suitable centrifuge device.

The device 20 further comprises a suitable reservoir 24 for accommodating buffer, such as PMI which are used during the process of preparing a biological material for scaffold seeding. The tip cutter 26 may be used for opening the otherwise closed syringe 29a of the mixing and seeding module 29. The prepared cells are seeded using the syringe 29a onto a scaffold sample 27a provided on a displaceable scaffold support table 27. Preferably, the scaffold support table is rotatable and comprises a plurality of nests for accommodating different scaffold samples.

Figure 3 presents in a schematic way an embodiment of a shear cutter which may be used in the cartilage cell processing device according to an aspect of the invention. Figure 3 in view 30a depicts schematically a cartilage preparation module comprising a cutter/digester 21, a MNC isolation module 22 and a mixing/seeding device 29. In accordance with the invention suitable respective biological material is being automatically transferred between these modules of the device according to the invention, as discussed with reference to Figure 2.

View 30b depicts schematically an embodiment of the cutting/digesting unit, wherein cutting of the cartilage is carried out using shear blades. Using shear blades has an advantage that a substantially equally sized pieces of the cartilage are provided. In order to improve release of the cartilage from the blades a specific coating may be provided thereon.

View 30c depicts schematically an embodiment of a blender which may be suitable to be used in the cutting/digesting unit 21. The blender may be provided with a blade manufactured from a plastics material. More details on the blender are presented with reference to Figure 4a.

Figure 4a presents a schematic view of an embodiment of a blender 40 of the cartilage cutting and digesting module 21 discussed with reference to Figure 2, which may be used in the cartilage cell processing device according to the invention.

The blender 40 comprises an input fluid port 41 and for supplying enzyme and /or washing fluid into the inner volume of the blender. The inner volume of the blender may be about 5 ml. Views 40a and 40b depict schematically a cross-section of the blender along a horizontal line. The blade 44 is motorized and is preferably adapted with an angulated tip having a longer portion 42a and a shorter portion 42b. It will be appreciated that the blade 44 may be a substantially flat body, or, alternatively it may have a shape of a propeller.

In view 40a a first mode of operation is depicted, wherein the blade is moving with the longer portion 42a as a leading end. In this case the blade will exert a cutting action on the pieces of cartilage 43 and provide a suitable manner to cut the cartilage pieces for optimal digestion.

In view 40b a second mode of operation of the blade 44 is depicted, when the blade is moving with its shorter side 42b as the leading end. Because there is a space between the shorter end 42b of the blade and the wall 42 of the cutter/digester, which space is comparable with the dimensions of the cartilage pieces 43, such mode of the blade's rotation will exert a massaging action on the cartilage.

As a result, when the first mode and the second mode are alternated an improvement of the cartilage may be achieved due to such alternating mechanical action on the cartilage material.

Figure 5 presents schematically an embodiment of the MNC isolation module for use in the cartilage cell processing device according to the invention. The MNC module according to an aspect of the invention comprises a reservoir 51, which is connected using suitable conduits 51a, 51b, 51c, 51d to different modules and/or parts of the device as discussed with reference to Figure 2.

In a preferred embodiment of the MNC isolation module, it is connected to an affinity filter 52. The conduit 51b may be used for supplying bone marrow to the MNC isolation module 51. The conduit 51d may be optional for additionally removing red cells using a washing step. A suitable release fluid may be supplied to the affinity filter 52 from a connected reservoir 53. When the MNC cells are isolated, the waste may be discharged using a reservoir 54. The isolated and harvested MNC may be supplied using the conduit 51c together with the release fluid supplied from the reservoir 53 to the mixing and seeding device 29, as is described with reference to Figure 2. The MNC module may be connected to a suitable cell counter, as is discussed with reference to the foregoing.

Figure 6 presents in a schematic way an embodiment of a scaffold cell mixing and seeding device. The cell mixing and seeding module 60 is preferable a module comprises disposable parts. Although in the context of the device as depicted in Figure 2 a fully automated transport of the biological material between the respective modules of the device is envisaged, it is also possible that only the mixing and seeding device is fully automated. Preferably, the mixing and seeding device comprises a centrifuge for providing the cell mixture in a disposable syringe 62. It is found to be advantageous to provide the disposable syringe 62, as it simplified application of the prepared cell seeded scaffold to a patient. Preferable, prior to scaffold seeding the scaffold material is hydrated. More preferably, the mixing and seeding device is adapted to on-line monitor the cell count and to provide a warning signal in the event there are not enough cells for a pre-determined scaffold, for example for a 18 mm ∅ scaffold. Such warning is advantageous as the surgeon can make a proper decision whether to proceed with the pre-determined (18mm) scaffold or to abort the procedure. In this way, cell number is allowing a surgeon to provide a patient with an effective cartilage repair implant. The syringe 62 may be advantageously provided with a breakable tip 62a, which m ay be removed from the syringe when the cells are deposited there within. It will be appreciated that the length of the syringe and the angle of its inclination with respect to the body of the mixing and seeding device may be optimized for achieving a substantially full deposition of the biological material inside the syringe. This is found to be advantageous to further minimize undesirable waste of the valuable biological material.

It will be appreciated that the modules as discussed with reference to Figures 3 - 6 may either form an integral part of the device according to the invention , or may alternatively, be provided as removable and/or disposable items. It will be further appreciated that the invention relates to both the individual modules, as described with reference to the foregoing, or to the device comprising these modules.

Although specific reference may have been made above to the use of embodiments of the invention in the context of cartilage cell processing, it will be appreciated that the invention may be used in other applications as well.

## Claims

1. An automated or semi-automated module-based cartilage cell processing device for preparing a cell seeded carrier from a biological material, comprising at least a cartilage preparation module, a cell isolation module, a cell mixing module and a carrier cell seeding module, wherein the cell mixing module and the carrier cell seeding module are integrated to provide a single unit;
wherein the cartilage preparation module comprises a blender comprising a blade which is rotatable in two directions, each rotating direction causing a substantially unique effect of the blade on the cartilage cells.

2. The module-based cartilage cell processing device according to claim 1, wherein the biological material is transported automatically between said modules.

3. The module-based cartilage cell processing device according to claim 1 or 2, wherein the cell seeded carrier comprises a scaffold, a gel, or is based on a further implantable carriers selected from a group consisting of: PEGT/PBT polymers, esterified hyaluronic acid scaffolds, fibrin glue, alginate, hyaluronic acid, PLGA, PEG based scaffolds or gels, Polyacrylamide carriers, Dextran based carriers and derivatives thereof.

4. The module-based cartilage cell processing device according to claim 1 or 2, wherein the cell isolation module is adapted to operate using the mononuclear cells (MNC) or the multipotent cells.

5. The module-based cartilage processing device according to claims 1 or 2, wherein the biological material comprises red blood cells, the cell isolation module is adapted to operate using a method to lyse the red blood cells, preferably using NH₄Cl.

6. The module-based cartilage cell processing device according to claim 1, further comprising a cutter.

7. The module-based cartilage cell processing device according to claim 6, wherein the cutter is a shear cutter comprising a blade.

8. The module-based cartilage cell processing device according to claim 6, wherein the cutter is adapted to push cartilage through a grid of blades or cutting wires.

9. The module-based cartilage cell processing device according to claim 1, wherein the blender is further adapted to combine a cutting step with a gyration or whirling step.

10. The module-based cartilage cell processing device according to claim 1 wherein the cell isolation module comprises a filter having a release buffer and/or a centrifugation unit.

11. The module-based cartilage cell processing device according to claim 10, wherein the cell seeding module is adapted to measure a volume of the cell suspension in order not to overflow the carrier using a counter or measuring device.

12. The module-based cartilage cell processing device according to claim 11, wherein the counter is operable using a principle selected from: a bulk through flow, a slide sample or a sample through flow.

13. The module-based cartilage cell processing device according to claim 1 or 2, wherein the carrier cell seeding module integrated with the mixing module comprises at least a partially disposable syringe or a disposable cell concentration tip.

14. The module-based cartilage cell processing device according to any one of the preceding claims, wherein at least one of the modules comprises antibiotics.

15. The module-based cartilage cell processing device according to any one of the preceding claims, wherein the respective modules are replaceable and/or disposable.

16. A cartilage preparation module for use in a module-based cartilage cell processing device according to claim 1 for preparing a cell seeded carrier from biological material, wherein the cartilage preparation module comprises a blender comprising a blade which is rotatable in two directions, each rotating direction causing a substantially unique effect of the blade on the cartilage cell.

17. The cartilage preparation module according to claim 16, further comprising a cutter wherein the cutter is a shear cutter comprising a blade.

18. The cartilage preparation module according to claim 16, wherein the blender is further adapted to combine a cutting step with a gyration or whirling step.

19. A method of cartilage cell processing comprising the following sequence of automated or semi-automated steps:
- cartilage cell preparation;
- cell isolation;
- cell mixing using the prepared cartilage cells and the isolated cells;
- cell seeding on a carrier using said mixture,
wherein cell mixing and cell seeding is carried out in an automated or semi-automated module-based cartilage cell processing device of claim 1.

## Patentansprüche

1. Automatisierte oder halbautomatisierte modulbasierte Knorpelzellen-Verarbeitungsvorrichtung zur Herstellung eines mit Zellen besäten Trägers aus einem biologischen Material, umfassend mindestens ein Knorpelvorbereitungsmodul, ein Zellenisolationsmodul, ein Zellenmischmodul und ein Trägerzellen-Aussaatmodul, wobei das Zellenmischmodul und Trägerzellen-Aussaatmodul zu einer Einheit integriert sind;
wobei das Knorpelvorbereitungsmodul einen Mischer umfasst, der eine Klinge umfasst, die in zwei Richtungen drehbar ist, wobei jede Drehrichtung eine im Wesentlichen einzigartige Wirkung der Klinge auf die Knorpelzellen verursacht.

2. Modulbasierte Knorpelzellen-Verarbeitungsvorrichtung nach Anspruch 1, wobei das biologische Material automatisch zwischen den Modulen transportiert wird.

3. Modulbasierte Knorpelzellen-Verarbeitungsvorrichtung nach Anspruch 1 oder 2, wobei der mit Zellen besäte Träger ein Gerüst, ein Gel umfasst oder auf einem weiteren implantierbaren Träger basiert, der aus einer Gruppe ausgewählt ist, bestehend aus: PEGT-/PBT-Polymeren, veresterten Hyaluronsäuregerüsten, Fibrinkleber, Alginat, Hyaluronsäure, PLGA-, PEGbasierten Gerüsten oder Gelen, Polyacrylamidträgern, Dextran-basierten Trägern und Derivaten davon.

4. Modulbasierte Knorpelzellen-Verarbeitungsvorrichtung nach Anspruch 1 oder 2, wobei das Zellenisolationsmodul angepasst ist, um unter Verwendung der mononukleären Zellen (MNC) oder der multipotenten Zellen zu arbeiten.

5. Modulbasierte Knorpel-Verarbeitungsvorrichtung nach Anspruch 1 oder 2, wobei das biologische Material rote Blutkörperchen umfasst, und das Zellisolationsmodul dazu angepasst ist, unter Verwendung eines Verfahrens zum Lysieren der roten Blutkörperchen, vorzugsweise unter Verwendung von NH₄Cl, zu arbeiten.

6. Modulbasierte Knorpelzellen-Verarbeitungsvorrichtung nach Anspruch 1, ferner umfassend einen Schneider.

7. Modulbasierte Knorpelzellen-Verarbeitungsvorrichtung nach Anspruch 6, wobei der Schneider ein Scherschneider mit einer Klinge ist.

8. Modulbasierte Knorpelzellen-Verarbeitungsvorrichtung nach Anspruch 6, wobei der Schneider dazu ausgelegt ist, Knorpel durch ein Gitter von Klingen oder Schneiddrähten zu drücken.

9. Modulbasierte Knorpelzellen-Verarbeitungsvorrichtung nach Anspruch 1, wobei der Mischer ferner angepasst ist, um einen Schneideschritt mit einem Rotations- oder Wirbelschritt zu kombinieren.

10. Modulbasierte Knorpelzellen-Verarbeitungsvorrichtung nach Anspruch 1, wobei das Zellenisolationsmodul einen Filter mit einem Freisetzungspuffer und/oder einer Zentrifugationseinheit umfasst.

11. Modulbasierte Knorpelzellen-Verarbeitungsvorrichtung nach Anspruch 10, wobei das Zellen-Aussaatmodul angepasst ist, um unter Verwendung eines Zählers oder einer Messvorrichtung ein Volumen der Zellsuspension zu messen, um den Träger nicht zu überlaufen.

12. Modulbasierte Knorpelzellen-Verarbeitungsvorrichtung nach Anspruch 11, wobei der Zähler unter Verwendung eines Prinzips betätigt werden kann, das ausgewählt ist aus: einem Massendurchfluss, einer Objektträgerprobe oder einem Probendurchfluss.

13. Modulbasierte Knorpelzellen-Verarbeitungsvorrichtung nach Anspruch 1 oder 2, wobei das in das Mischmodul integrierte Trägerzellen-Aussaatmodul mindestens eine teilweise wegwerfbare Spritze oder eine wegwerfbare Zellenkonzentrationsspitze umfasst.

14. Modulbasierte Knorpelzell-Verarbeitungsvorrichtung nach einem der vorhergehenden Ansprüche, wobei mindestens eines der Module Antibiotika umfasst.

15. Modulbasierte Knorpelzellen-Verarbeitungsvorrichtung nach einem der vorhergehenden Ansprüche, wobei die jeweiligen Module austauschbar und/oder wegwerfbar sind.

16. Knorpelvorbereitungsmodul zur Verwendung in einer modulbasierten Knorpelzell-Verarbeitungsvorrichtung nach Anspruch 1 zur Herstellung eines mit Zellen besäten Trägers aus biologischem Material, wobei das Knorpelvorbereitungsmodul einen Mischer umfasst, der eine Klinge umfasst, die in zwei Richtungen drehbar ist, wobei jede Drehrichtung eine im Wesentlichen einzigartige Wirkung der Klinge auf die Knorpelzellen verursacht.

17. Knorpelvorbereitungsmodul nach Anspruch 16, ferner umfassend einen Schneider, wobei der Schneider ein Scherschneider ist, der eine Klinge umfasst.

18. Knorpelvorbereitungsmodul nach Anspruch 16, wobei der Mischer ferner angepasst ist, um einen Schneideschritt mit einem Rotations- oder Wirbelschritt zu kombinieren.

19. Verfahren zur Verarbeitung von Knorpelzellen, umfassend die folgende Abfolge von automatisierten oder halbautomatisierten Schritten:
- Vorbereitung der Knorpelzellen;
- Zellenisolation;
- Vermischen der Zellen unter Verwendung der vorbereiteten Knorpelzellen und der isolierten Zellen;
- Zellenaussaat auf einem Träger unter Verwendung der Mischung, wobei das Vermischen von Zellen und das Aussäen von Zellen in einer automatisierten oder halbautomatisierten modulbasierten Knorpelzellen-Verarbeitungsvorrichtung nach Anspruch 1 durchgeführt werden.

## Revendications

1. Dispositif de traitement automatisé ou semi-automatisé de cellules de cartilage basé sur des modules pour préparer un porteur ensemencé par des cellules à partir d'un matériel biologique, comprenant au moins un module de préparation de cartilage, un module d'isolement de cellules, un module de mélange de cellules et un module d'ensemencement de cellules sur un porteur, dans lequel le module de mélange de cellules et le module d'ensemencement de cellules sur un porteur sont intégrés pour fournir une seule unité ;
dans lequel le module de préparation de cartilage comprend un mélangeur comprenant une pale qui peut tourner dans deux directions, chaque direction de rotation provoquant un effet sensiblement unique de la pale sur les cellules de cartilage.

2. Dispositif de traitement de cellules de cartilage basé sur des modules selon la revendication 1, dans lequel le matériel biologique est transporté automatiquement entre lesdits modules.

3. Dispositif de traitement de cellules de cartilage basé sur des modules selon la revendication 1 ou 2, dans lequel le porteur ensemencé par des cellules comprend un support, un gel, ou est basé sur des porteurs implantables supplémentaires sélectionnés dans le groupe consistant en : les polymères PEGT/PBT, les supports à base d'acide hyaluronique estérifié, une colle à base de fibrine, un alginate, l'acide hyaluronique, un PLGA, les supports ou gels à base de PEG, les porteurs à base de polyacrylamide, les porteurs à base de dextrane et leurs dérivés.

4. Dispositif de traitement de cellules de cartilage basé sur des modules selon la revendication 1 ou 2, dans lequel le module d'isolement de cellules est adapté pour fonctionner en utilisant les cellules mononucléaires (CMN) ou les cellules multipotentes.

5. Dispositif de traitement de cartilage basé sur des modules selon la revendication 1 ou 2, dans lequel le matériel biologique comprend des érythrocytes, le module d'isolement de cellules est adapté pour fonctionner en utilisant une méthode pour lyser les érythrocytes, de préférence en utilisant NH₄Cl.

6. Dispositif de traitement de cellules de cartilage basé sur des modules selon la revendication 1, comprenant en outre un coupeur.

7. Dispositif de traitement de cellules de cartilage basé sur des modules selon la revendication 6, dans lequel le coupeur est une cisaille comprenant une lame.

8. Dispositif de traitement de cellules de cartilage basé sur des modules selon la revendication 6, dans lequel le coupeur est adapté pour pousser un cartilage à travers une grille de lames ou de fils de coupe.

9. Dispositif de traitement de cellules de cartilage basé sur des modules selon la revendication 1, dans lequel le mélangeur est adapté en outre pour combiner une étape de découpe avec une étape de giration ou de tourbillonnement.

10. Dispositif de traitement de cellules de cartilage basé sur des modules selon la revendication 1, dans lequel le module d'isolement de cellules comprend un filtre ayant un tampon de libération et/ou une unité de centrifugation.

11. Dispositif de traitement de cellules de cartilage basé sur des modules selon la revendication 10, dans lequel le module d'ensemencement de cellules est adapté pour mesurer un volume de la suspension de cellules afin de ne pas engorger le porteur au moyen d'un compteur ou d'un dispositif de mesure.

12. Dispositif de traitement de cellules de cartilage basé sur des modules selon la revendication 11, dans lequel le compteur est utilisable en utilisant un principe sélectionné parmi : un écoulement en vrac, un échantillon sur lame ou un écoulement d'échantillon.

13. Dispositif de traitement de cellules de cartilage basé sur des modules selon la revendication 1 ou 2, dans lequel le module d'ensemencement de cellules sur un porteur intégré au module de mélange comprend au moins une seringue partiellement jetable ou une pointe de concentration de cellules jetable.

14. Dispositif de traitement de cellules de cartilage basé sur des modules selon l'une quelconque des revendications précédentes, dans lequel au moins l'un des modules comprend des antibiotiques.

15. Dispositif de traitement de cellules de cartilage basé sur des modules selon l'une quelconque des revendications précédentes, dans lequel les modules respectifs sont remplaçables et/ou jetables.

16. Module de préparation de cartilage pour son utilisation dans un dispositif de traitement de cellules de cartilage basé sur des modules selon la revendication 1 pour préparer un porteur ensemencé par des cellules à partir d'un matériel biologique, dans lequel le module de préparation de cartilage comprend un mélangeur comprenant une pale qui peut tourner dans deux directions, chaque direction de rotation provoquant un effet sensiblement unique de la pale sur les cellules de cartilage.

17. Module de préparation de cartilage selon la revendication 16, comprenant en outre un coupeur dans lequel le coupeur est une cisaille comprenant une lame.

18. Module de préparation de cartilage selon la revendication 16, dans lequel le mélangeur est adapté en outre pour combiner une étape de découpe avec une étape de giration ou de tourbillonnement.

19. Méthode de traitement de cellules de cartilage comprenant la séquence suivante d'étapes automatisées ou semi-automatisées :
- la préparation de cellules de cartilage ;
- l'isolement de cellules ;
- un mélange de cellules en utilisant les cellules de cartilage préparées et les cellules isolées ;
- l'ensemencement de cellules sur un porteur en utilisant ledit mélange,
dans laquelle le mélange de cellules et l'ensemencement de cellules sont réalisés dans un dispositif de traitement automatisé ou semi-automatisé de cellules de cartilage basé sur des modules selon la revendication 1.
